Europäisches Patentamt

European Patent Office

Office européen des brevets

(11) Publication number: **0 376 872**
**A2**

# (12) EUROPEAN PATENT APPLICATION

(21) Application number: **89500127.9**

(22) Date of filing: **13.12.89**

(51) Int. Cl.⁵: **A61N 1/08, A61N 1/40**

(30) Priority: **28.12.88 ES 8803980**

(43) Date of publication of application:
**04.07.90 Bulletin 90/27**

(84) Designated Contracting States:
**AT BE DE FR GB IT NL SE**

(71) Applicant: **INDIBA, S.A.**
**Ronda del Guinardo 64**
**E-08025 Barcelona(ES)**

(72) Inventor: **Calbet Benach, José**
**c/o Indiba, S.A. Ronda del Guinardó 64**
**E-08025 Barcelona(ES)**
Inventor: **Jodra Hernandez, Epifanio**
**c/o Indiba, S.A. Ronda del Guinardó 64**
**E-08025 Barcelona(ES)**

(74) Representative: **Pastells Teixido, Manuel**
**c/o PASTELLS TEIXIDO, S.L. Pau Claris 138,**
**5-1a**
**E-08009 Barcelona(ES)**

(54) Electronic device for controlling medical therapy apparatus.

(57) An electronic device for controlling capacitive transfer medical therapy apparatus comprising an insulated metallic active electrode which is applied by movement over the patient. The new device enhances the efficiency of these apparatus without having to move the active electrode which is situated statically on the affected area of the patient, for which purpose the device comprises an oscillator circuit which is inserted in the apparatus's main high frequency circuit thereby modifying the electrical resistance across two points of such main circuit to achieve an intermittent output signal at preset intervals, said oscillator circuit being preferably connected to the main circuit power regulator control.

## Electronic Device for controlling medical therapy apparatus

This invention relates to an electronic device for controlling medical therapy apparatus which operate by capacitive transfer. The device is distinguished by its efficacious use and for the improved performance it provides to the models of this type of apparatus.

This device is applied mainly to the so-called CAPACITIVE TRANSFER high frequency (0.5-1 MHz) apparatus, for application to certain parts of the human body so as to produce therein thermal effects by the passage of currents crossing through the epidermal zone capacitively. The frequency and intensity of the oscillations in question may be regulated, within certain limits, to adjust the generator operating conditions to the characteristic of the body regions affected.

However, the therapeutical radiation in the above apparatus as used in medicine and in beauty treatments is generally produced by continuous wave devices, i.e., there are no breaks or pauses, since the circuits operate their production cycles uninterruptedly, i.e. with a higher or lower intensity, but without changing or modifying the continuity of the oscillations of the currents applied to the body.

After substantial research, evidence has been obtained that treatments with these methods are much more effective if they are applied intermittently, i.e. varying the duration and intensity of the electric field generated in the treated area. This would be attributable to the heat generation mechanisms in the cells and of other physical effects as a result of inducing the high frequency cur rents corresponding to the oscillations applied in the human body tissue.

The device of the invention produces oscillations at a rate involving variations of intensity (at frequencies of 2 to 10 cycles per second) which may mean attenuation or even suppression of the radiations with time, with preset on and off periods thereof alternating at the application electrode.

The way of providing for the above described operation is by modifying the electrical resistance across two points of the therapeutical apparatus circuit, the magnitude being directly related, preferably, with that of a potentiometer forming part of the circuit itself, which allows the oscillations generation and application mode to be adjusted.

The advantages of having the apparatus operate as described are mainly the following: the body electrode may be left attached to the part of interest, unlike present practice, which requires the electrode to be moved, to avoid the risk of causing irritation to the affected parts. The locally generated temperature level changes automatically, without having to move the electrode, thereby preventing discomfort and other undesired effects for the user.

The device described is really an improvement of an electronic regenerator operating by capacitive power transfer, such as the one described in Spanish utility models 287.964 and 8800658. The use of the said apparatus has proved that it is only fully effective when there are variations of hyperthermia in the body regions treated, and thus the need to move the applicator electrode about.

Hereafter there is given an explanation of why better results are obtained with the incorporation of the new very low frequency oscillator circuit in the regenerator main circuit.

Based on the operation of a capacitor, it is known that, as an intrinsic characteristic, the start up current (intensity) is much higher than during normal operation, since the loads and discharges during normal operation are continuous about 800,000 times per second, whereby there is no time materially for an overload to pass at any time. Nevertheless, the mere fact of interrupting the output signal for 200 milliseconds four times per second provides more than sufficient time for an overload to flow making the penetration much deeper and thereby providing better spectacular results.

To facilitate the explanation, the description is accompanied by a sheet of drawings showing one embodiment which is given as a non-limiting example of the scope of the present invention.

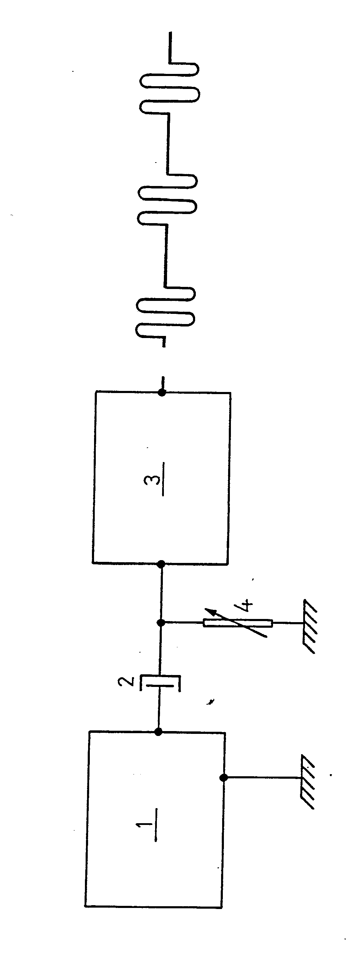

In the drawing.

The sole figure is a block diagram of the fundamental stages of a device of the type described, as well as of certain components associated with the stages.

Stage 1 is a very low frequency (2 to 8 cycles per second) oscillator circuit. The capacitor 2 is used to facilitate the passage of the pulses corresponding to this frequency to the ends of the potentiometer or control 4 regulating the apparatus output power. The output signal is interrupted at each pulse as many times as indicated by the stage 1 circuit frequency setting, as shown by the output wave form of the block 3, corresponding to the general amplifier circuit.

The effect produced by each pulse of stage 1 is that the resistance or impedance across the ends of the regulator or potentiometer 4 is reduced to a minimum value, i.e. if the potentiometer set at point 8 (of the ten the apparatus is provided with) has, for example, 1,000 ohms, it will be brought down to 10 or 30 ohms when it receives the pulses, the power being reduced to zero.

The said electric effect occurs, in stage or block 3, by alternate biasing and debiasing of a semi-conductor member, by varying its internal electrical resistance from minimum to maximum, said magnitude having an influence on that of the potentiometer 4.

Within the essence thereof, the invention may be embodied in other forms differing only in detail from the exemplary embodiment described, which forms will equally be covered by the scope claimed.

## Claims

1.- An electronic device for controlling capacitive transfer medical therapy apparatus of the type comprising an insulated metallic active electrode for application thereof, by movement, on the affected parts of a patient, characterised in that it comprises an oscillator circuit inserted in any appropriate point on the apparatus's main high frequency circuit so as to modify the electrical resistance across two points of such circuit and achieve intermittent interruption at preset intervals several times per second of the high frequency output signal or wave, thereby achieving higher apparatus performance without having to move the active applicator electrode about on the patient's body.

2.- The electronic device for controlling medical therapy apparatus of claim 1, characterised in that the oscillator circuit is preferably connected to the main apparatus circuit at the power regulator control for said main circuit whereby the generation and application mode of the electrical oscillations may thus be regulated.

EP 0 376 872 A2